(19) Europäisches Patentamt
European Patent Office
Office européen des brevets

(11) Publication number: **0 112 297**
**B1**

(12) **EUROPEAN PATENT SPECIFICATION**

(45) Date of publication of patent specification: **29.10.86**

(21) Application number: **83810602.9**

(22) Date of filing: **19.12.83**

(51) Int. Cl.$^4$: **C 07 C 149/20,**
C 07 C 149/00, C 07 C 148/00,
C 07 D 307/32, D 21 H 3/12

(54) **Process for the preparation of bis(perfluoroalkyl-alkylthio)alkonoic acids and lactone derivatives thereof.**

(30) Priority: **23.12.82 US 452531**

(43) Date of publication of application:
**27.06.84 Bulletin 84/26**

(45) Publication of the grant of the patent:
**29.10.86 Bulletin 86/44**

(84) Designated Contracting States:
**BE CH DE FR GB IT LI**

(56) References cited:
**EP-A-0 073 732**
**US-A-4 239 915**

**BULLETIN DE LA SOCIETE CHIMIQUE DE FRANCE, no. 3, March 1973, pages 1037-1042, Paris, FR S. DUCHER et al.: "Action de thiols en milieux acide ou basique sur quelques gamma-lactones éthyléniques"**

(73) Proprietor: **CIBA-GEIGY AG**
**Klybeckstrasse 141**
**CH-4002 Basel (CH)**

(72) Inventor: **Falk, Robert A.**
**35 Glenside Drive**
**New City, NY 10956 (US)**

**EP 0 112 297 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to a process for the preparation of bis(perfluoroalkyl-alkylthio)alkanoic acids and lactone derivatives thereof.

Bis(perfluoroalkyl-alkylthio)alkanoic acids of the formula

(1) 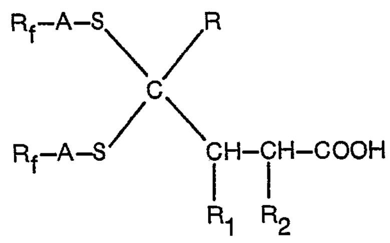

wherein each $R_f$ is independently perfluoroalkyl of 3 to 18 carbon atoms or perfluoroalkoxyperfluoroalkyl of 3 to 18 carbon atoms, each A is independently alkylene of up to 6 carbon atoms or said alkylene interrupted by —O—, —N(H)— or —N(lower alkyl)—, $R_1$ is hydrogen or lower alkyl, $R_2$ is halogen, lower alkyl, aryl or aryl substituted lower alkyl, and R is hydrogen or lower alkyl, have been found to be highly advantageous in the form of the ammonium and amine salts thereof, especially in the alkanolamine salts, such as the diethanolamine salt thereof forms aqueous solutions and emulsions which are useful in rendering natural and synthetic polyamide materials, such as leather and polyamide textile materials, and cellulosic materials, such as paper articles, hydrophobic and oleophobic.

Acids of the formula (1) are described in US 4,239,915. The preparation of these acids according to said patent specification comprises the reaction of the $R_f$-thiols of the formula $R_fA$—SH with aldehydo or keto acids of the formula O=CR—A—COOH in the presence of acids.

It is now one object of the instant invention to provide a new process for the preparation of these bis-(perfluoroalkyl-alkylthio)alkanoic acids of the formula (1).

It is a further object of the instant invention to provide novel perfluoroalkyl-alkylthio lactone intermediates. These and other objects of the invention are apparent from the following detailed description.

According to the present invention, the compounds of the formula (1) are prepared by reacting a perfluoroalkyl-alkylmercaptan of the formula

(2) $R_f$—A—SH

wherein $R_f$ and A are as defined above, with a lactone of the formula

(3) 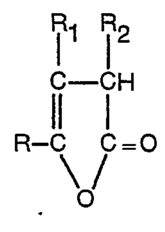

where R, $R_1$ and $R_2$ are as defined above, in the presence of a catalytic amount of a Lewis acid, to form a perfluoroalkyl-alkylthio lactone of the formula

(4) 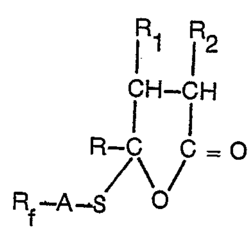

wherein $R_f$, A, R, $R_1$ and $R_2$ are as defined above, and reacting said perfluoroalkyl-alkylthio lactone, with or without isolation thereof, with additional perfluoroalkyl-alkylmercaptan of the formula

(2a) $R_f$—A—SH

wherein $R_f$ and A are as defined above, in the presence of a catalytic amount of a Lewis acid.

The $R_f$ group in the compounds of the formula (1) and (4) preferably contains 3 to 18 carbon atoms. It may be straight or branched chain and can be represented for example by perfluorinated propyl, butyl, pentyl, hexyl, octyl, decyl, undecyl, dodecyl, tetradecyl, hexadecyl and octadecyl as well as isomers thereof, further by said perfluorinated groups which are interrupted by an oxygen atom such as for example $F_7C_3—O—CF_2—(CF_2)_{0-5}$, which is a preferred perfluoroalkoxyperfluoroalkyl group. More preferably, $R_f$ is perfluoroalkyl of 4 to 16 carbon atoms. Advantageously, the $R_f$ group may be a mixture of such perfluoroalkyl groups.

The linking group A is an alkylene group having for example up to 6 carbon atoms. Alkylene groups having 2 to 4 carbon atoms and especially ethylene are the preferred linking groups. Said alkylene groups A can be interrupted by —O—, —N(H)— or —N(lower alkyl)— moieties, where lower alkyl denotes those alkyl radicals having 1 to 6 carbon atoms such as methyl, ethyl, propyl, butyl, pentyl or hexyl. Preferably, such interrupted alkylene groups contain an oxygen atom or a $—N(CH_3)—$ group such as $—(CH_2)_{1-4}—O—(CH_2)_{1-4}$ or $—(CH_2)_{1-4}—N(CH_3)—(CH_2)_{1-4}—$. $R_1$ is hydrogen or one of the lower alkyl groups mentioned above. Preferably, $R_1$ is hydrogen or methyl and most preferably hydrogen.

$R_2$ has the meanings assigned to $R_1$. Additionally, $R_2$ denotes aryl. Preferred aryl groups are phenyl and substituted phenyl radicals such as for example phenyl substituted by lower alkyl as defined above, lower alkoxy, lower alkanoyl and lower alkanoyloxy (where "lower" indicates that said radicals contain 1 to 6 carbon atoms) such as tolyl, methoxyphenyl or dimethoxyphenyl, further phenyl substituted by halogen such as chlorine or bromine. $R_2$ also denotes aryl substituted lower alkyl (having 1 to 6 carbon atoms) and is preferably phenylethyl or benzyl. More preferably, $R_2$ is hydrogen, methyl, ethyl or benzyl, and most preferably $R_2$ is hydrogen.

In preferred compounds of the formula (1), A is alkylene of 2 to 4 carbon atoms, $R_1$ is hydrogen and $R_2$ is hydrogen, methyl, ethyl or benzyl. In more suitable compounds of the formula (1), A is ethylene and $R_2$ is hydrogen.

R is preferably hydrogen or lower alkyl as defined above. Preferably R denotes alkyl of 1 to 4 carbon atoms, and is most preferably methyl.

The lactones of formula (3) belong to a known class of compounds, and many have been prepared by the dehydration of the corresponding keto acids, viz.:

(3) 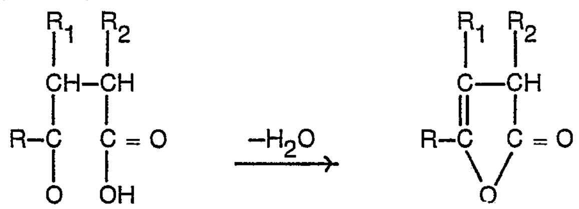


by a variety of techniques, including slow dehydration, or by dehydration in the presence of an acidic dehydrating agent, such as phosphoric acid, acetyl chloride, acetic anhydride-sulfuric acid mixtures, acetic anhydride, and the like. One advantageous procedure is to dehydrate the keto acid by the use of acetic anhydride and Amberlyst 15 catalyst according to the procedure set forth in J. Het. Chem., Vol. 12, No. 4, pages 749—754 (1975).

As stated above, the reactions between the mercaptan of formula (2) and the lactone of formula (3), and the reaction between the mercaptan of formula (2a) and the substituted lactone of formula (4), are advantageously conducted in the presence of a catalytic amount of a Lewis acid. Suitable Lewis acids include the mineral acids, such as hydrochloric and sulfuric acid, organic acids such as acetic acid, para toluene sulfonic acid and the like, acid clays and resins, and boron trifluoride, or the diethyl ether complex thereof. Thus, the amount by weight of catalyst based on the total weight of the starting materials can vary between broad limits, e.g. between about 0.005% to about 10% by weight, preferably between about 0.01% to about 2% by weight.

Moreover, representative lactones of formula (3) and alternate synthetic methods for their preparation e.g. from beta, gamma dibromo acids, substituted propargylmalonic acids, and the like, are known in the art, as see Y. Shyamsunder Rao, Chem. Rev., Vol. 46, No. 4, pages 353—388 (1964).

The perfluoroalkyl-alkyl mercaptans of formula (2) and (2a) may be the same or different and commercially available conventional mixtures of perfluoroalkyl-alkyl mercaptans may be used.

In one embodiment, the perfluoroalkyl-alkylthio lactone of formula (4) may be isolated. If it is desired to isolate the compound of formula (4), the mole ratio of the mercaptan of the formula (2) to the lactone of formula (3) is desirably between about (0.1—1.2):1, preferably between about (0.5—1):1.

The perfluoroalkyl-alkylthio lactone of formula (4) may be then separated from the reaction mixture by conventional techniques, such as precipitation and filtration. For example, if the reaction is conducted in the presence of an inert solvent, such as benzene, toluene, chlorobenzene, methoxybenzene or the like, the reaction mixture may be cooled, and optionally a substantial non solvent for the compound of formula (4) such as an alkane, e.g. heptane, is added to promote precipitation. The precipitated compound of formula (4) may then be isolated by separation from the liquid mixture, such as by filtration, decantation, and the like. In the second step, the compound of the formula (4) is then reacted with the compound of the formula (2a) in a mole ratio of 1:(1—1.6) or preferably, 1:(1.1—1.2), in order to obtain the compound of the formula (1).

In an alternate embodiment, the perfluoroalkyl-alkylthio lactone of formula (4) need not be isolated, but is further reacted by the addition of the perfluoroalkyl-alkyl mercaptan of formula (2a). Desirably, sufficient additional perfluoroalkylalkyl mercaptan of formula (2a) is added such that the total mole ratio of mercaptan (formula (2) plus (2a)) added to the reaction mixture based on the amount of the lactone of formula (3) is between about (1.3—2.4):1, preferably about (1.6—2.2):1, to obtain the product of formula (1).

In another alternate embodiment, where it is not desired to isolate the perfluoroalkyl-alkylthio lactone of formula (4), then the reaction to obtain the product of formula (1) may be conducted in one step, by the addition of the requisite mole ratio of mercaptan to lactone as set forth in the preceding paragraph.

The reaction temperature for the process, whether conducted in two steps, or in one step, can be between about −20°C and 120°C, preferably between about 0°C and 60°C.

The reactions between the compounds of formula (2) and formula (3), and formula (4) and formula (2a), respectively, are advantageously conducted in a substantially liquid phase, i.e. as a suspension or solution, irrespective of whether the process is conducted in one or two steps.

Thus, where one or more of the starting materials are liquid, under the reaction conditions the reaction may be conducted in the presence or absence of an inert liquid solvent or diluent. Where all of the starting materials are solid and do not form a liquid eutectic mixture under the reaction conditions, an inert solvent or diluent is advantageously employed. The nature of the solvent or diluent is not critical, but should not interfere with the desired reaction. Suitable solvents and diluents include aliphatic and aromatic hydrocarbons which are unsubstituted or substituted with non-reactive substituents, such as benzene, toluene, chlorobenzene, dichlorobenzene, methoxybenzene, methylene chloride, xylene, decane, dipropyl ether, acetic acid, and the like. In order to assist in driving the reaction to completion, it is additionally contemplated to use a solvent wherein the desired final product, e.g. the product of formula (1), is substantially less soluble or insoluble in the reaction medium and wherein the starting materials are relatively more soluble, such as toluene, ethyl benzene, hexane, and the like.

The conversion of the compounds of the formula (1) into ammonium or amine salts such as alkanolamine salts, and the formation of solutions or emulsions of these salts occur by processes well known in the art, i.e. reaction of the compounds of the formula (1) with a compound of the formula

(5) 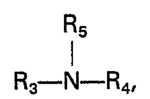

wherein $R_3$, $R_4$ and $R_5$ are hydrogen or alkyl or hydroxyalkyl each having 1 to 4 carbon atoms, and preparing solutions or emulsions of the compounds thus obtained of the formula

(6) 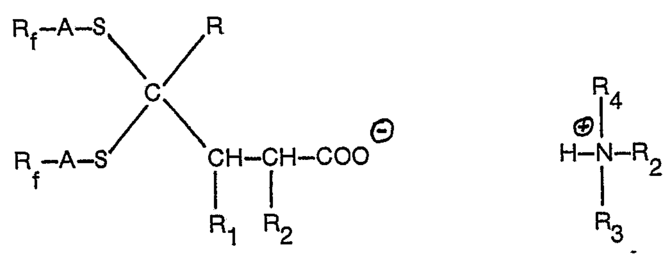 .

It is evident, that the process according to the present invention is applicable for a series of bis(perfluoroalkyl-alkylthio)alkanoic acids wherein the substituents R, $R_f$, $R_1$, $R_2$ and the linking group A have meanings different from the inventively ones.

The following Examples are set forth for illustrative purposes only and are not intended to limit the scope of the invention. All parts are by weight unless otherwise stated.

Example 1

4-hydroxy-4-[2-(n-heptadecafluorooctyl)ethylthio]-pentanoic acid gamma-lactone.

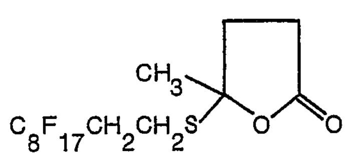

15 g (0.15 mol) of α-4-hydroxy-3-pentenoic acid gamma-lactone, 250 ml of toluene, and 48 g (0.1 mol) of 2-(n-perfluorooctyl)ethyl mercaptan are added to a reaction vessel. There is then added a small amount (about 0.5 g) of p-toluene sulfonic acid, and the solution is stirred for about 16 hours at ambient temperature. The mixture is cooled to 0°C, stirred for 1 hour and filtered cold. The resulting precipitate is then washed with a small amount of cold toluene (about 10 ml) and dried in a partial vacuum at 40°C. 34 g

of 4-hydroxy-4-[2-(n-heptadecafluorooctyl)ethylthio]-pentanoic acid gamma-lactone are obtained in 96% purity by gas-liquid chromatography.

Analysis for $C_{15}H_{11}F_{17}O_2S$:

| | | | | |
|---|---|---|---|---|
| Found: | C, 30.6; | H, 1.9; | F, 53.0; | S, 5.6 |
| Calc.: | C, 31.2; | H, 1.9; | F, 55.9; | S, 5.6 |

The mass spectrum was consistent with the structure of the product. The molecular ion ($M^{\oplus}$) was not present, but ions of m/z 534 ($M^{\oplus}$—$CO_2$) and m/z 99 ($M^{\oplus}$—$C_8F_{17}CH_2CH_2S$) were observed.

## Example 2

4,4-Bis-[2-(n-perfluoroalkyl)ethylthio]-pentanoic acid $(R_fCH_2CH_2S)_2C(CH_3)CH_2CH_2COOH$

5.0 g (0.05 mol), of 4-hydroxy-3-pentenoic acid gamma-lactone, 140 ml of toluene, 55.6 g (0.1 mol) of 2-(n-perfluoroalkyl)ethyl mercaptan of the formula $R_f$—$CH_2CH_2SH$, wherein present $R_f$ is $C_6/C_8/C_{10}/C_{12}/C_{14}$ = 3/29/47/17/3, is charged into a 500 ml reaction vessel and heated to 40°C. Then, approximately 0.5 ml of boron trifluoride etherate 1:1 complex is added and the reaction mixture is stirred for two hours under a nitrogen atmosphere blanket while maintaining a reaction temperature of 40—45°C. The reaction mixture is then cooled to 0—5°C and stirred for one hour. The product, 4,4-bis-[2-(n-perfluoroalkyl)ethylthio]pentanoic acid is obtained as a suspension with a conversion of 90%. The product is isolated from solution by adding about 50 ml of n-heptane at about 0°C, and recovering the product precipitate by filtration. It is a white solid having a melting point of 112—115°C.

## Example 3

4,4-Bis-[2-(n-perfluorooctyl)ethylthio]pentanoic acid $(C_8F_{17}CH_2CH_2S)_2C(CH_3)CH_2CH_2COOH$

5.0 g (0.05 mol) of 4-hydroxy-3-pentanoic acid gamma-lactone, 27.8 g (0.05 mol) of 2-(n-perfluorooctyl)ethyl mercaptan, 80 ml of toluene and 0.5 g of p-toluene sulfonic acid are charged under nitrogen to a reaction vessel. The reaction mixture is stirred for about 16 hours at approximately 25°C. A white suspension of 4-hydroxy-4-[2-(n-perfluorooctyl)ethylthio]-pentanoic acid gamma-lactone is obtained in 80—90% conversion. Then an additional 27.8 g (0.05 mol) of 2-(n-perfluorooctyl)-ethyl mercaptan, 70 ml of toluene and 0.5 g of boron trifluoride etherate is added to the reaction mixture suspension. The resulting mixture is stirred at a temperature of 40—45°C for two hours. After cooling to 0°C and stirring for an hour at ambient temperature the resulting white suspension is filtered and washed with toluene. The precipitate is then dried under reduced pressure at 40°C. The 4,4-Bis-[2-(n-perfluorooctyl)ethylthio]-pentanoic acid product is 99% pure by gas-liquid chromatography.

It is a white powder having a melting point of 106°C. The NMR-spectra was consistent with its structure.

## Example 4

4,4-Bis-[2-(n-perfluorobutyl)ethylthio]pentanoic acid $(C_4F_9CH_2CH_2S)_2C(CH_3)CH_2CH_2COOH$

2.1 g (0.021 mol) of 4-hydroxy-3-pentanoic acid gamma-lactone, 11.2 g of 2-(n-perfluorobutyl)ethyl mercaptan (0.04 mol), 30 ml toluene and about 0.5 ml of boron trifluoride etherate 1:1 complex, is charged into a reaction vessel at ambient temperature (approx. 20°C), which is followed by a slight exotherm to about 29°C. After about 22 hours at room temperature (approx. 20°C) the solution is diluted with about 30 ml n-heptane and is cooled to about 0°C. The resulting suspension is filtered and the precipitate is washed with heptane and dried in a vacuum. The resulting product, 4,4-bis-[2-(n-perfluorobutyl)ethylthio]-pentanoic acid is obtained in 86% purity, as confirmed by NMR analysis. (Assignments (δ-values): 1.58, s (3 protons) $CH_3$; 1.77—3.33 br.complex (12 protons) $CH_2$; 10.90, s. (exchangeable proton) OH. It has a melting point of 65°C.

Using the methods according to Examples 1 to 4, the compounds listed in the following Table are prepared.

T a b l e

| Mercaptan of the formula (2) | Mercaptan of the formula (2a) | Lactone of the formula (3) | Perfluorolactone of the formula (4) | Bisperfluoro-alkanoic acid of the formula (1) |
|---|---|---|---|---|
| $(CF_3)_2CHOCF_2(CH_2)_3SH$ | $(CH_3)_2CFOCF_2(CH_2)_3SH$ | 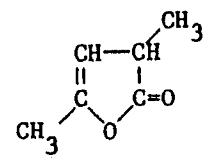 | 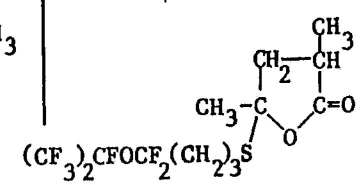 | 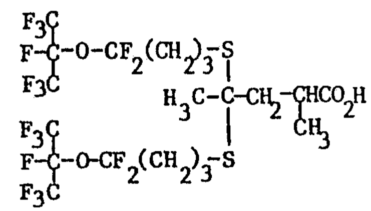 |
| $C_6F_{13}CH_2CH_2SH$ | $C_4H_9CH_2SH$ | 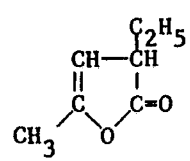 | 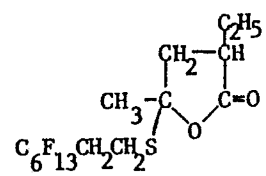 | 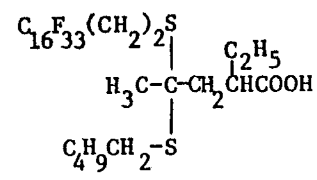 |
| $C_8F_{17}CH_2CH_2O(CH_2)_3SH$ | $C_8F_{17}CH_2CH_2O(CH_2)_3SH$ | 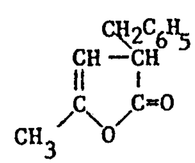 | 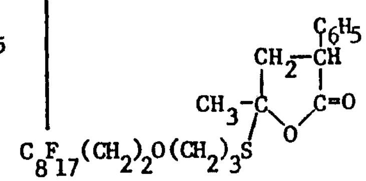 | 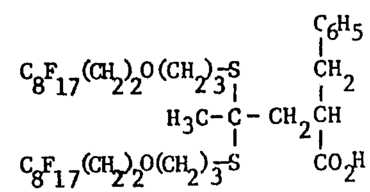 |

T a b l e (continuation)

| Mercaptan of the formula (2) | Mercaptan of the formula (2a) | Lactone of the formula (3) | Perfluorolactone of the formula (4) | Bisperfluoro-alkanoic acid of the formula (1) |
|---|---|---|---|---|
| $C_8F_{17}CH_2CH_2N(CH_3)CH_2CH_2SH$ | $C_8F_{17}CH_2CH_2N(CH_3)CH_2CH_2SH$ | 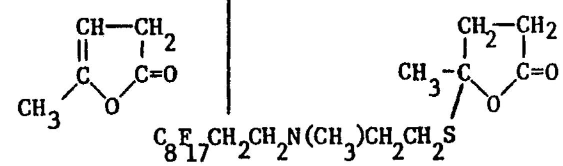 | | 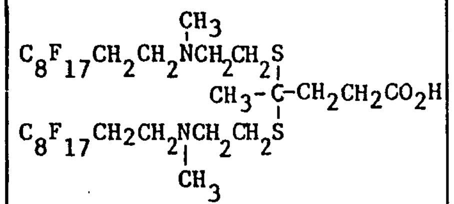 |
| $C_6F_{13}CH_2CH_2SH$ | $C_6F_{13}CH_2CH_2SH$ | 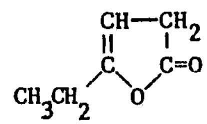 | 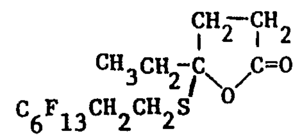 | 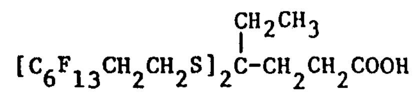 |
| $C_6F_{13}CH_2CH_2SH$ | $C_6F_{13}CH_2CH_2SH$ | 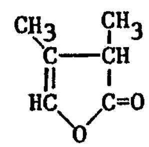 | 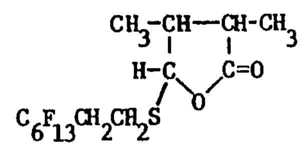 | 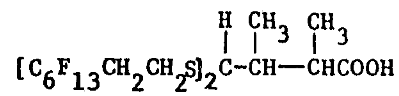 |
| $C_6D_{13}CH_2CH_2SH$ | $C_6F_{13}CH_2CH_2SH$ | 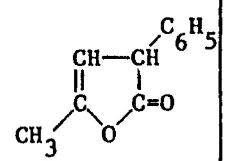 | 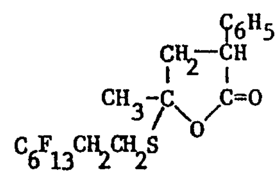 | 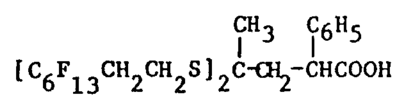 |

**Claims**

1. A process for the preparation of bis(perfluoroalkyl-alkylthio)alkanoic acids of the formula

(1) 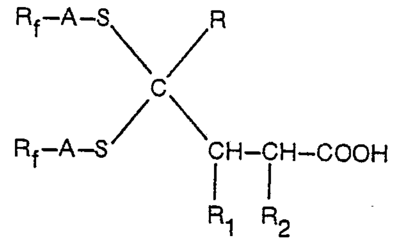

wherein each $R_f$ is independently perfluoroalkyl of 3 to 18 carbon atoms or perfluoroalkoxyperfluoroalkyl of 3 to 18 carbon atoms, each A is independently alkylene of up to 6 carbon atoms or said alkylene interrupted by —O—, —N(H)— or N(lower alkyl)—, $R_1$ is hydrogen or lower alkyl, $R_2$ is hydrogen, lower alkyl, aryl or aryl substituted lower alkyl, and R is hydrogen or lower alkyl, by reacting a perfluoroalkyl-alkyl mercaptan of the formula

(2) $R_f$—A—SH

wherein $R_f$ and A are as defined above, with a lactone of the formula

(3) 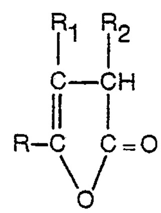

where R, $R_1$ and $R_2$ are as defined above, in the presence of a catalytic amount of a Lewis acid, to form a perfluoroalkyl-alkylthio lactone of the formula

(4) 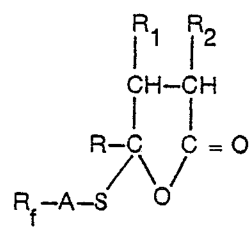

wherein $R_f$, A, R, $R_1$ and $R_2$ are as defined above, and reacting said perfluoroalkyl-alkylthio lactone, with or without isolation thereof, with additional perfluoroalkyl-alkylmercaptan of the formula

(2a) $R_f$—A—SH

wherein $R_f$ and A are as defined above, in the presence of a catalytic amount of a Lewis acid.

2. A process according to claim 1, wherein $R_f$ is perfluoroalkyl of 4 to 16 carbon atoms.

3. A process according to claim 2, wherein A is alkylene of 2 to 4 carbon atoms, $R_1$ is hydrogen or methyl and $R_2$ is hydrogen, methyl, ethyl or benzyl.

4. A process according to claim 3, wherein A is ethylene and $R_2$ is hydrogen.

5. A process according to claim 1, wherein R is hydrogen or alkyl of 1 to 4 carbon atoms.

6. A process according to claim 1, wherein the mercaptan of formula (2) is identical to the mercaptan of formula (2a).

7. A process according to claim 1, wherein the process is conducted by a one step addition of the mercaptan of formula (2) and the mercaptan of formula (2a) to the lactone of formula (3).

8. A process according to claim 7, wherein the total mole ratio of mercaptan added to the lactone of formula (3) is between about (1.3—2.4):1.

9. A process according to claim 1, which comprises reacting in a first step 0.1 to 1.2 moles of the compound of the formula (2) with 1 mole of the compound of the formula (3), isolating the compound of the formula (4) and further reacting the compound of the formula (4) in a second step with the compound of the formula (2a) in the molar ratio of 1:(1—1.6).

10. A process according to claim 1, wherein the reactions between the compounds of the formula (2) and (3) and formula (4) and (2a) is conducted in a substantially liquid phase.

11. A process according to claim 1, which is carried out between −20 and 120°C.

12. A perfluoroalkyl-alkylthio lactone of the formula

(4) 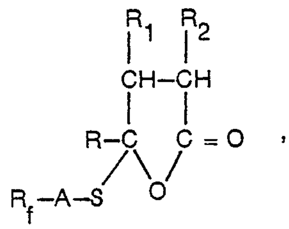 ,

wherein $R_f$ is perfluoroalkyl of 3 to 18 carbon atoms or perfluoroalkoxyperfluoroalkyl of 3 to 18 carbon atoms, A is alkylene of 1 to 6 carbon atoms or said alkylene interrupted by —O—, —N(H)— or —N(lower alkyl)—, $R_1$ is hydrogen or lower alkyl, $R_2$ is hydrogen, lower alkyl, aryl or aryl substituted lower alkyl, and R is hydrogen or lower alkyl.

13. A compound according to claim 12, wherein $R_f$ is perfluoroalkyl of 4 to 16 carbon atoms.

14. A compound according to claim 12, wherein A is alkylene of 2 to 4 carbon atoms, $R_1$ is hydrogen or methyl and $R_2$ is hydrogen, methyl, ethyl or benzyl.

15. A compound according to claim 12, wherein A is ethylene and $R_2$ is hydrogen.

16. A compound according to claim 12, wherein R is hydrogen or alkyl of 1 to 4 carbon atoms.

17. Use of a compound of the formula (4) for the preparation of a compound of the formula (1).

**Patentansprüche**

1. Verfahren zur Herstellung von Bis-(perfluoralkylalkylthio)alkylcarbonsäuren der Formel (1)

(1) 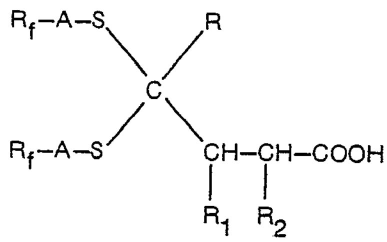

worin jedes $R_f$ unabhängig Perfluoralkyl mit 3 bis 18 C-Atomen oder Perfluoralkoxyperfluoralkyl mit 3 bis 18 C-Atomen ist, jedes A unabhängig gegebenenfalls durch —O—, —NH— oder —N(Niederalkyl)— unterbrochenes Alkylen mit bis zu 6 C-Atomen ist, $R_1$ Wasserstoff oder Niederalkyl bedeutet, $R_2$ Wasserstoff, Niederalkyl oder gegebenenfalls mit Niederalkyl substituiertes Aryl darstellt und R Wasserstoff oder Niederalkyl ist, dadurch gekennzeichnet, dass man ein Perfluoralkylalkylmerkaptan der Formel (2)

(2) $R_f$—A—SH,

worin $R_f$ und A wie zuvor definiert sind, mit einem Lakton der Formel (3)

(3) 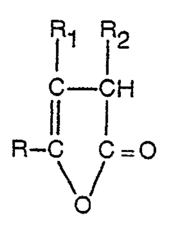 ,

worin R, $R_1$ und $R_2$ wie zuvor definiert sind, in Gegenwart einer katalytischen Menge einer Lewissäure zu einem Lakton der Formel (4)

(4) 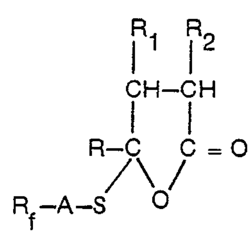

umsetzt, worin $R_f$, A, R, $R_1$ und $R_2$ wie zuvor definiert sind, und dieses Lakton gegebenenfalls nach vorheriger Isolierung, in Gegenwart einer katalytischen Menge einer Lewissäure mit weiterem Perfluoralkylalkylmerkaptan der Formel 2a

(2a) $R_f—A—SH$,

worin $R_f$ und A wie zuvor definiert sind, umsetzt.

2. Verfahren gemäss Anspruch 1, worin $R_f$ für Perfluoralkyl mit 4 bis 16 C-Atomen steht.

3. Verfahren gemäss Anspruch 2, worin A $C_2$—$C_4$-Alkylen ist und $R_1$ Wasserstoff oder Methyl und $R_2$ Wasserstoff, Methyl, Ethyl oder Benzyl bedeuten.

4. Verfahren gemäss Anspruch 3, worin A Ethylen und $R_2$ Wasserstoff sind.

5. Verfahren gemäss Anspruch 1, worin R Wasserstoff oder $C_1$—$C_4$-Alkyl ist.

6. Verfahren gemäss Anspruch 1, worin die Merkaptane der Formeln (2) und (2a) identisch sind.

7. Verfahren gemäss Anspruch 1, worin die Merkaptane der Formeln (2) und (2a) in einer Verfahrensstufe mit dem Lakton der Formel (3) umgesetzt werden.

8. Verfahren gemäss Anspruch 7, worin das Molverhältnis von Merkaptanen zum Lakton der Formel (3) zwischen (1,3—2,4):1 beträgt.

9. Verfahren gemäss Anspruch 1, bei dem zunächst 0,1 bis 1,2 Mol des Mercaptans der Formel (2) mit einem Mol des Laktons der Formel (3) umgesetzt werden und danach die Verbindung der Formel (4) isoliert wird, und die Verbindung der Formel (4) in einer zweiten Verfahrensstufe mit einem Merkaptan der Formel (2a) im Moverhältnis von 1:(1—1,6) umgesetzt wird.

10. Verfahren gemäss Anspruch 1, worin die Reaktion der Verbindungen der Formeln (2) und (3) bzw. Formeln (4) und (2a) in flüssiger Phase durchgeführt wird.

11. Verfahren gemäss Anspruch 1, dadurch gekennzeichnet, dass es zwischen −20 bis 120°C durchgeführt wird.

12. Perfluoralkylalkylthiolaktone der Formel (4)

(4) 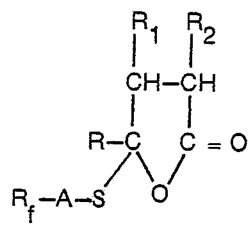 ,

worin $R_f$ Perfluoralkyl oder Perfluoralkoxyperfluoralkyl mit 3 bis 18 C-Atomen, A gegebenenfalls durch —O—, —N(H) oder —N(Niederalkyl)— unterbrochenes $C_1$—$C_6$-Alkylen, $R_1$ Wasserstoff oder Niederalkyl, $R_2$ Wasserstoff, Niederalkyl oder gegebenenfalls durch Niederalkyl substituiertes Aryl und R Wasserstoff oder Niederalkyl sind.

13. Verbindung gemäss Anspruch 12, worin $R_f$ $C_4$—$C_{16}$-Perfluoralkyl ist.

14. Verbindung gemäss Anspruch 12, worin A $C_2$—$C_4$-Alkylen, $R_1$ Wasserstoff oder Methyl und $R_2$ Wasserstoff, Methyl, Ethyl oder Benzyl sind.

15. Verbindung gemäss Anspruch 12, worin A Ethylen und $R_2$ Wasserstoff sind.

16. Verbindung gemäss Anspruch 12, worin R Wasserstoff oder $C_1$—$C_4$-Alkyl ist.

17. Verwendung von Verbindungen der Formel (4) zur Herstellung von Verbindungen der Formel (1).

**Revendications**

1. Un procédé de préparation de bis(perfluoroalkyl-alkylthio)alcanoïque de formule:

(1) 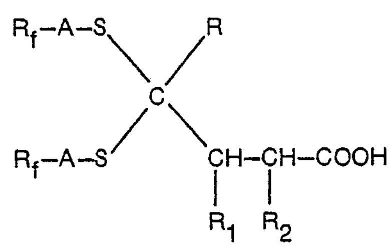

(dans laquelle chaque $R_f$ est, indépendamment de l'autre, un perfluoroalkyle en $C_3$ à $C_{18}$ ou un perfluoroalcoxyperfluoroalkyle en $C_3$ à $C_{18}$, chaque A est, indépendamment de l'autre, un alkylène pouvant avoir jusqu'à 6 atomes de carbone et pouvant être éventuellement interrompu par —O—, —N(H)— ou un groupe —N(alkyle inférieur)—, $R_1$ est l'hydrogène ou un alkyle inférieur, $R_2$ un halogène, un alkyle inférieur, un aryle ou un alkyle inférieur substitué par un aryle, et R l'hydrogène ou un alkyle inférieur), par réaction d'un perfluoroalkyl-alkyl mercaptan de formule:

$R_f$—A—SH (2)

avec une lactone de formule:

(3) 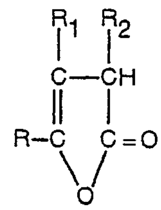

en présence d'un acide de Lewis comme catalyseur, pour former une perfluoroalkyl-alkylthio-lactone de formule:

(4) 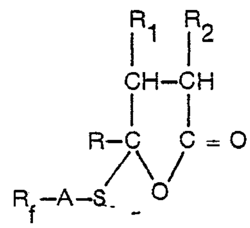

que l'on fait ensuite réagir, sans l'avoir isolée ou après l'avoir isolée, avec une nouvelle quantité d'un perfluoroalkyl-alkyl mercaptan de formule:

$R_f$—A—SH (2a)

en présence d'un acide de Lewis comme catalyseur.

2. Un procédé selon la revendication 1 dans lequel, dans les formules indiquées, $R_f$ est un perfluoroalkyle en $C_4$ à $C_{16}$.

3. Un procédé selon la revendication 2 dans lequel A est un alkylène en $C_2$ à $C_4$, $R_1$ l'hydrogène ou un méthyle et $R_2$ l'hydrogène ou le groupe méthyle, éthyle ou benzyle.

4. Un procédé selon la revendication 3 dans lequel A est le groupe éthylène et $R_2$ l'hydrogène.

5. Un procédé selon la revendication 1 dans lequel R est l'hydrogène ou un alkyle en $C_1$ à $C_4$.

6. Un procédé selon la revendication 1 dans lequel le mercaptan de formule (2) est identique à celui de formule (2a).

7. Un procédé selon la revendication 1 réalisé en une seule étape par addition du mercaptan de formule (2) et du mercaptan de formule (2a) à la lactone de formule (3).

8. Un procédé selon la revendication 7 dans lequel le rapport molaire total du mercaptan ajouté à la lactone de formule (3) est compris entre 1,3 et 2,4.

9. Un procédé selon la revendication 1 dans lequel on fait réagir dans une première étape 0,1 à 1,2 mole du composé de formule (2) avec 1 mole du composé de formule (3), on isole le composé de formule (4) formé puis on le fait réagir dans une seconde étape avec le composé de formule (2a) dans un rapport molaire de 1:(1—1,6).

10. Un procédé selon la revendication 1 dans lequel les réactions entre les composés de formules (2) et (3) et de formules (4) et (2a) se font en phase liquide.

11. Un procédé selon la revendication 1 qui est effectué entre −20 et 120°C.

12. Perfluoroalkyl-alkylthio-lactones de formule:

(4) 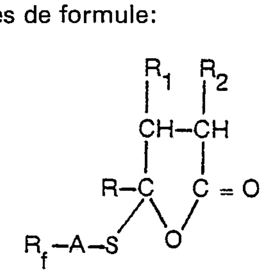

dans laquelle $R_f$ désigne un perfluoroalkyle en $C_3$ à $C_{18}$ ou un perfluoroalcoxyperfluoroalkyle en $C_3$ à $C_{18}$, A un alkylène en $C_1$ à $C_6$ pouvant être éventuellement interrompu par —O—, —N(H)— ou un groupe —N(alkyle inférieur)—, $R_1$ l'hydrogène ou un alkyle inférieur, $R_2$ l'hydrogène, un alkyle inférieur, un aryle ou un alkyle inférieur substitué par un aryle, et R l'hydrogène ou un alkyle inférieur.

13. Un composé selon la revendication 12 dans lequel $R_f$ est un perfluoroalkyle en $C_4$ à $C_{16}$.

14. Un composé selon la revendication 12 dans lequel A est un alkylène en $C_2$ à $C_4$, $R_1$ l'hydrogène ou un méthyle et $R_2$ l'hydrogène ou le groupe méthyle, éthyle ou benzyle.

15. Un composé selon la revendication 12 dans lequel A est le groupe éthylène et $R_2$ l'hydrogène.

16. Un composé selon la revendication 12 dans lequel R est l'hydrogène ou un alkyle en $C_1$ à $C_4$.

17. L'emploi des composés de formule (4) selon la revendication 12 pour la préparation de composés de formule (1) selon la revendication 1.